# FASCICULE DE BREVET EUROPEEN

(11) **EP 3 586 796 B1**
(45) Date de publication et mention de la délivrance du brevet: **05.08.2026**
(21) Numéro de dépôt: 19182195.8
(22) Date de dépôt: 25.06.2019
(51) Int. Cl.: A61F 2/14

(54) **IMPLANT ORBITAL COMPORTANT DES FENÊTRES TRAVERSANTES POUR SUTURES**
ORBITAIMPLANTAT, DAS DURCHGEHENDE FENSTER FÜR NÄHTE UMFASST
ORBITAL IMPLANT COMPRISING DUAL-ASPECT WINDOWS FOR SUTURES

(30) Priorité: 29.06.2018 FR 1870778
(43) Date de publication de la demande: 01.01.2020
(73) Titulaire: France Chirurgie Instrumentation SAS, 75015 Paris (FR)
(72) Inventeur: Madec, Aurélie, 25480 Ecole-Valentin (FR)
(74) Mandataire: Eidelsberg, Olivier Nathan

(56) Documents cités:
- WO-A1-2006/034565
- SU-A1- 603 385
- US-B2- 8 118 867

## Description

La présente invention se rapporte à un implant orbital sensiblement en forme de bille destiné à remplacer un globe oculaire après énucléation et/ou éviscération.

On connaît déjà de l'art antérieur, et notamment de US 8 118 867, un implant orbital comportant une bille réalisée en un matériau poreux destinée à remplir la cavité orbitale et couplée, du côté postérieur ou arrière, à un élément sensiblement en forme de demi-coque, qui recouvre en partie la partie postérieure de la bille. La demi-coque comporte des fenêtres traversantes pour le passage de fils de suture des muscles oculomoteurs. Cette demi-coque est réalisée en un matériau dégradable non-poreux, tandis que la bille est réalisée en un matériau poreux non-dégradable, par exemple en hydroxyapatite poreuse.

Ces implants orbitaux de l'art antérieur présentent plusieurs inconvénients. Ils sont difficiles à implanter. En outre, ils présentent un taux élevé d'échec ou rejet de l'implantation, qui nécessite souvent de recommencer l'opération plusieurs fois.

La présente invention vise à surmonter les inconvénients de l'art antérieur en proposant un implant orbital sensiblement en forme de bille, qui est plus facile à implanter et qui présente notamment un taux d'échec ou de rejet plus faible, voire nul.

Au document WO 2006/034565, il est décrit un assemblage suivant le préambule de la revendication 1. Dans cet assemblage, les fils de suture passent dans des tunnels 18 mais pas dans les cheminées 20a, 20b.

Suivant un premier aspect de l'invention, un assemblage est tel que défini à la revendication 1, des perfectionnements étant définis aux sous revendications 2 à 9.

Le premier aspect de l'invention se rapporte aussi à un implant orbital de forme sensiblement sphérique destiné à remplacer un globe oculaire après énucléation et/ou éviscération est constitué d'un corps de base en un matériau poreux, notamment qui favorise la colonisation par l'organisme, et un élément postérieur ou arrière à fenêtres traversantes recouvrant une partie du corps de base, l'élément postérieur comportant une pluralité de fenêtres traversantes pour le passage de fils de suture pour la suture des muscles oculomoteurs dans et hors de l'espace défini entre le corps de base et l'élément postérieur, et en définissant pour l'implant de forme sensiblement sphérique un repère passant par son centre, un axe vertical Z correspondant à l'axe antérieur-postérieur et deux axes XY dans le plan équatorial, repère par rapport auquel on définit les angles de latitude et de longitude d'un point quelconque à la surface de l'implant, l'angle de longitude du pôle le plus postérieur ou arrière, dit pôle supérieur, étant de 90° tandis que l'angle de longitude d'un point à l'équateur étant de 0°, est caractérisé en ce que l'élément à fenêtre comprend au moins une première fenêtre traversante inférieure, qui s'étend sur une première plage inférieure de longitudes, et au moins une deuxième ouverture supérieure, qui s'étend sur une deuxième plage supérieure de longitudes, et il existe au moins un méridien s'étendant de l'équateur au pôle qui traverse ou passe par les deux fenêtres inférieure et supérieure, la ou les fenêtre(s) de traversée inférieure(s) ayant une forme oblongue ou allongée, le ou les bords inférieurs étant le ou les bords en longueur et s'étendant le long d'un bord périphérique de la surface extérieure du corps de base.

De préférence, la plage inférieure de longitudes et la plage supérieure de longitudes sont distinctes l'une de l'autre, à savoir ne se chevauchent pas, par exemple la première plage étant égale à [60°,70°] et la deuxième plage étant égale à [75°, 85°].

En prévoyant ainsi de disposer au moins deux fenêtres inférieure et supérieure pour permettre le passage de fils de suture alternativement par exemple suivant un premier sens par la fenêtre inférieure dans l'espace défini entre le corps de base et l'élément à fenêtres, puis par la fenêtre supérieure hors dudit espace suivant un autre sens ou inversement mais qui peut être dans la même direction que le premier sens, on améliore grandement pour le chirurgien l'ergonomie d'introduction des fils de suture, qu'il peut réaliser ainsi suivant une direction qui notamment correspond à un méridien.

Suivant encore un autre aspect de l'invention constituant en soi une invention qui peut être mis en oeuvre indépendamment de chacun des deux autres aspects ci-dessus, mais aussi en combinaison avec l'un et/ou l'autre des deux aspects précédents, un implant orbital de forme sensiblement sphérique destiné à remplacer un globe oculaire après énucléation et/ou éviscération est constitué d'un corps de base en un matériau poreux, notamment qui favorise la colonisation par l'organisme, et un élément postérieur ou arrière à fenêtres traversantes recouvrant une partie du corps de base, l'élément postérieur comportant une pluralité de fenêtres traversantes pour le passage de fils de suture pour la suture des muscles oculomoteurs dans et hors de l'espace défini entre le corps de base et l'élément postérieur, est caractérisé en ce que au moins une fenêtre de traversée, notamment la au moins une fenêtre supérieure, a une forme triangulaire, dont une pointe est orientée vers le pôle supérieur de l'implant.

Prévoir une telle géométrie d'au moins une fenêtre, notamment de plusieurs fenêtres, en particulier des fenêtres supérieures, donne au chirurgien une grande facilité pour l'insertion et l'extraction des fils de suture dans et hors de l'espace entre l'élément à fenêtres et le corps de base.

De préférence, le bord périphérique le long duquel s'étendent les bords inférieurs des fenêtres inférieure est défini par une pluralité de tronçons en arc de cercle, notamment quatre, répartis de manière uniforme angulairement le long de la périphérie, chaque arc de cercle ayant sa concavité tournée vers le haut, et une pluralité correspondante, notamment quatre, de tronçons de bord formant méplats, notamment horizontaux rectilignes, disposés en alternance avec les tronçons en arc de cercle.

De préférence, la section ou aire transversale, mesurée perpendiculairement à l'axe Z, de au moins une fenêtre inférieure est plus grande que la section ou aire transversale de la au moins une fenêtre supérieure qui lui est associée.

De préférence la bille formant le corps de base est une sphère tronquée sur le dessus, notamment suivant un plan de coupe parallèle au plan équatorial, notamment sur une plage de longitudes comprises entre 60° à 70° et 90°.

De préférence, l'élément postérieur en forme de plaque est concave avec sa concavité tournée vers le bas, notamment en forme de calotte sphérique, notamment en ayant un rayon de courbure sensiblement égal au rayon de la partie sphérique de la sphère tronquée.

Suivant un mode de réalisation préféré, les deux matériaux respectivement pour le corps de base et l'élément postérieur, sont à base d'une même matière, notamment thermoplastique, notamment le polyéthylène (PE), notamment le Polyéthylène Haute Densité (PEHD) ou le Polyéthylène de masse molaire très élevée (UHMWPE), mais en ayant des grains de tailles différentes, notamment compris entre 1,0 mm et 1,5 mm pour le corps de base et inférieur à 1,1 mm pour l'élément postérieur.

Suivant un autre aspect de l'invention, constituant en soi une invention pouvant être mise en œuvre indépendamment des autres aspects de l'invention, un assemblage comporte au moins un fil de suture et un implant orbital de forme sensiblement sphérique destiné à remplacer un globe oculaire après énucléation et/ou éviscération est constitué d'un corps de base en un matériau poreux, notamment qui favorise la colonisation par l'organisme, et un élément postérieur ou arrière à fenêtres traversantes recouvrant une partie du corps de base, l'élément postérieur comportant une pluralité de fenêtres traversantes dans lesquelles passe le au moins un fil de suture pour la suture des muscles oculomoteurs dans et hors de l'espace défini entre le corps de base et l'élément postérieur, est caractérisé en ce que l'élément postérieur à fenêtres est fixé au corps de base, au moins une fenêtre, de préférence une pluralité de fenêtres, notamment quatre fenêtres, ayant un bord respectif, notamment un bord inférieur respectif, au moins en partie défini et délimité par le corps de base.

En prévoyant ainsi d'une part de fixer l'élément à fenêtres au corps de base, notamment en forme de bille, en matériau poreux colonisable de manière à ce qu'au moins une partie de bord, notamment le bord inférieur de la ou des fenêtres, soit formée par le corps de base, on s'assure que le ou les muscles suturés sont bien en contact avec le bord de la fenêtre et la partie de la surface du corps de base sous la fenêtre et se coloniseront ainsi convenablement, contrairement à l'art antérieur où il était en contact avec la matière non-colonisable de la coque. Ainsi, la suture est mieux réalisée, la bille est plus stable en position finale et le taux de rejet de l'opération d'insertion est plus faible.

A titre d'exemple, on décrit maintenant un mode de réalisation préféré de l'invention en se reportant aux dessins, dans lesquels :

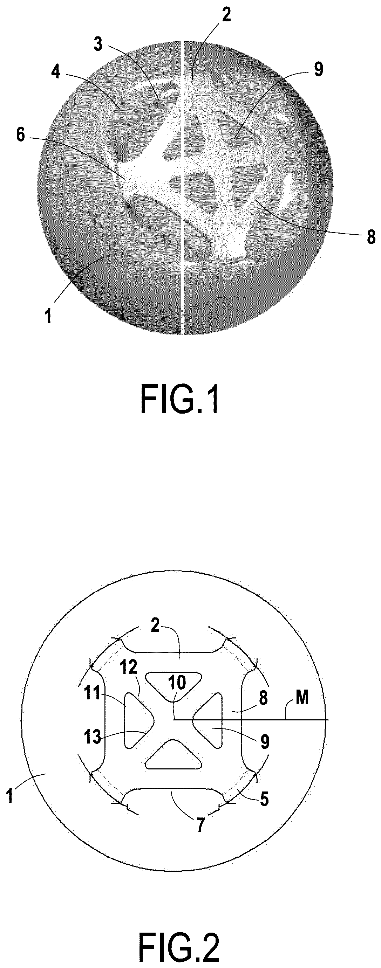

- La figure 1: est une vue en perspective de dessus d'un implant suivant un mode de réalisation de l'invention ;
- La figure 2: est une vue de dessus de l'implant de la figure 1 ;

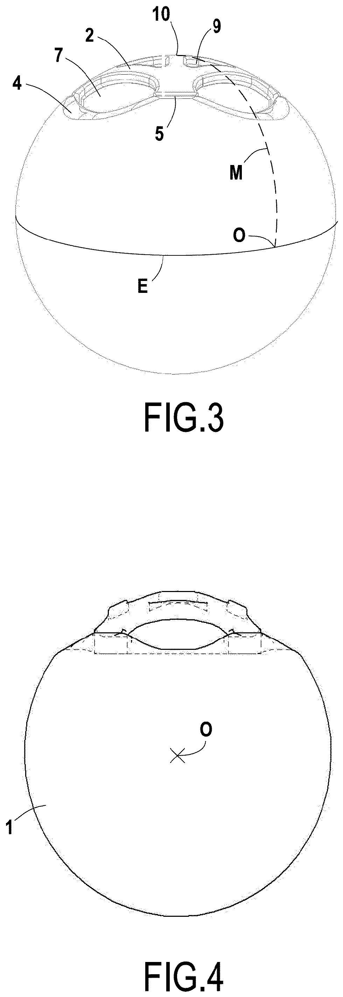

- La figure 3: est une vue en perspective de côté de l'implant des figures 1 et 2 ; et
- La figure 4: est une vue en coupe de la figure 1.

Aux figures, il est représenté un implant orbital suivant l'invention. L'implant comporte un corps 1 principal ou de base constitué d'une bille sensiblement en forme de sphère tronquée en un matériau poreux et biocompatible favorisant la colonisation cellulaire et donc d'une part la prise de la bille en position lorsqu'elle est implantée dans le globe oculaire, après énucléation et/ou éviscération, et d'autre part la prise des muscles oculomoteurs avec l'implant.

Dans la demande, on définit le point de l'implant destiné à être le plus à l'arrière ou postérieur comme étant le point le plus supérieur (comme représenté au figures) et l'axe vertical de bas en haut, notamment aux figures, est l'axe antérieur-postérieur ou avant-arrière.

La bille 1 est tronquée dans sa partie supérieure, notamment sur une plage de longitudes supérieures s'étendant de 60° à 90° environ. Il est défini sur le sommet de la bille tronquée une cavité 3 de fond plat parallèle au plan équatorial et creusée vers l'intérieur de la bille en étant délimitée par une paroi 4 latérale périphérique intérieure dont le bord supérieur est défini par quatre tronçons en arcs de cercle répartis de manière uniforme angulairement autour de la cavité, chaque arc de cercle s'étendant sur environ 80° à 85° en ayant sa concavité tournée vers le haut, et par quatre tronçons 5 de bord formant méplats horizontaux rectilignes disposés en alternance avec les tronçons en arc de cercle et qui s'étendent, mesuré le long du bord périphérique chacun sur environ 5 à 10°.

D'autre part, cet implant comporte un élément 2 en forme de plaque incurvée à fenêtres traversantes en un matériau non-poreux et de préférence colonisable. La plaque 2 est incurvée en ayant sa concavité tournée vers le bas et est fixée au corps 1 de base, notamment par surmoulage. Cette fixation par surmoulage s'effectue par quatre pattes 6 de la plaque au niveau des méplats 5 de la paroi latérale.

La plaque 2 est incurvée en ayant sa concavité tournée vers la cavité 3 de manière à être à distance du fond de la cavité délimitée par la paroi 4. La plaque 2 et le bord supérieur de la paroi 4 délimitent ensemble quatre fenêtres 7 traversantes inférieures de forme sensiblement oblongues ou allongée, dont les bords inférieurs respectifs de plus grande longueur sont définis par les tronçons de bords en arc de cercle du bord supérieur de la paroi 4, qui sont donc réparties de manière uniforme à la périphérie de la paroi 4, tandis que les bords 8 longs supérieurs, sensiblement rectilignes, sont délimités et définis par la plaque 2.

En outre, il est formé dans la plaque 2, entre les bords 8 supérieurs des fenêtres 7 inférieures et le pôle 10 supérieur ou postérieur, quatre fenêtres 9 supérieures en forme de triangles rectangles dont les sommets respectifs à 90° pointent chacun vers le pôle supérieur 10 postérieur. Chaque fenêtre 9 traversante comporte un bord 11 inférieur parallèle au bord 8 supérieur d'une fenêtre 7 inférieure respective et deux bords 12, 13 sensiblement rectilignes issus de ce bord 11 inférieur et se réunissant en le sommet à 90° respectif du triangle, à proximité du pôle 10.

Lorsque l'on trace un méridien M, à savoir un quart de cercle le long de la surface extérieure de l'implant entre le pôle P supérieur ou postérieur et le plan équatorial E de la bille qui coupe ou passe par une fenêtre 9 supérieure, alors ce méridien coupe ou passe également par une fenêtre 7 inférieure associée qui se trouve directement en dessous de la fenêtre 9 le long de ce méridien M. De préférence, la réciproque est également vraie.

Le rayon de la sphère, qui est également le rayon de la bille, peut être compris entre 5 mm et 12 mm.

Les dimensions des fenêtres inférieures sont telles qu'une plus grande dimension en longueur (mesurée dans la direction horizontale) peut être comprise entre 6 mm et 7 mm et une plus grande dimension en largeur (mesurée le long d'un méridien) peut être comprise entre 1,5 mm et 2,5 mm.

Les dimensions des fenêtres supérieures sont telles qu'une plus grande dimension en longueur (mesurée dans la direction horizontale) peut être comprise entre 3 mm et 4 mm et une plus grande dimension en largeur (mesurée le long d'un méridien) peut être comprise entre 2 mm et 2,5 mm.

Le rayon des tronçons de bord en arc de cercle du bord supérieur de la paroi 4 peut être compris entre 1 mm et 2 mm, tandis que la longueur (mesurée dans la direction horizontales) des méplats 5 intermédiaires faisant l'interface avec les pattes 6 de la plaque 2 peut être comprise entre 3 mm et 4 mm, et la hauteur de la paroi 4 dans la direction verticale au niveau de ces méplats peut être comprise entre 2 mm et 3 mm, de sorte que l'aire de l'interface de fixation entre une patte de la plaque et le corps de base peut être comprise entre 6 mm² et 12 mm².

Pour fabriquer l'implant, on peut notamment réaliser séparément l'élément 2 en forme de plaque et la bille tronquée, par exemple par moulage avec des moules de forme adaptés, puis on dispose les deux éléments dans un moule pour les mouler ensemble, avec interposition d'une pièce dont le volume correspond à l'espace que l'on souhaite obtenir entre les deux pièces.

Le corps de base peut notamment être en polyéthylène poreux haute densité, tandis que la plaque 2 à fenêtre peut être également en PEHD, mais cependant avec une taille de grain différente de la taille de grain du PEHD utilisé pour le premier élément. En effet on choisit un grain pour la bille 1 qui soit tel que le PEHD soit poreux, tandis que le grain du PEHD choisi pour la plaque 2 est tel que la plaque 2 n'est pas poreuse mais colonisable. Ainsi, lorsque l'on place l'ensemble de nouveau dans un moule et qu'on surmoule la plaque 2 à la bille 1, on obtient une bonne fixation par fusion des grains au niveau de l'interface pattes-méplats, les grains étant certes de tailles différentes mais présentant entre eux une bonne affinité des matières qui peuvent donc être bien liées intimement en contact l'une avec l'autre.

Bien évidemment, d'autres procédés de fabrication peuvent être prévus, et notamment d'autres procédés pour lier les deux éléments entre eux, par exemple par collage, soudure ou analogue.

En outre on peut choisir d'autres matériaux pour la bille comme pour la plaque, notamment pour la bille l'hydroxyapatite (HA), de préférence naturelle mais aussi synthétique, l'oxyde d'aluminium poreux, le zirconium poreux, le polyuréthane poreux et tout autre matériau poreux biocompatible capable de colonisation fibro vasculaire.

Pour la plaque, outre le polyéthylène non poreux, on peut aussi utiliser l'hydroxyapatite (HA), de préférence naturelle mais aussi synthétique, l'oxyde d'aluminium, le zirconium, le polyuréthane et tout autre matériau biocompatible.

## Revendications

1. Assemblage comportant un implant orbital de forme sensiblement sphérique destiné à remplacer un globe oculaire après énucléation et/ou éviscération et au moins un fil de suture, l'implant étant constitué d'un corps (1) de base en un matériau poreux, notamment qui favorise la colonisation par l'organisme, et un élément (2) postérieur ou arrière à fenêtres traversantes recouvrant une partie du corps de base, l'élément postérieur comportant une pluralité de fenêtres traversantes pour le passage de fils de suture pour la suture des muscles oculomoteurs dans et hors de l'espace défini entre le corps de base et l'élément postérieur, et en définissant pour l'implant de forme sensiblement sphérique un repère passant par son centre, un axe vertical Z correspondant à l'axe antérieur-postérieur et deux axes XY dans le plan équatorial, repère par rapport auquel on définit les angles de latitude et de longitude d'un point quelconque à la surface de l'implant, l'angle de longitude du pôle le plus postérieur ou arrière, dit pôle supérieur, étant de 90° tandis que l'angle de longitude d'un point à l'équateur étant de 0°, **caractérisé en ce que** l'élément (1) à fenêtre comprend au moins une première fenêtre (7) traversante inférieure, qui s'étend sur une première plage inférieure de longitudes, et au moins une deuxième fenêtre (9) supérieure, qui s'étend sur une deuxième plage supérieure de longitudes, et il existe au moins un méridien (M) s'étendant de l'équateur au pôle qui traverse ou passe par les deux fenêtres (7) inférieure et ( 9) supérieure.

2. Assemblage suivant la revendication 1, **caractérisé en ce que** la ou les fenêtre(s) (7) de traversée inférieures ont une forme oblongue ou allongée, un ou des bords inférieurs étant le ou les bords en longueur et s'étendant le long d'un bord périphérique de la surface extérieure du corps de base.

3. Assemblage suivant la revendication 2, **caractérisé en ce que** le bord périphérique le long duquel s'étendent les bords inférieurs des fenêtres de traversée inférieures est défini par une pluralité de tronçons en arc de cercle, notamment quatre, répartis de manière uniforme angulairement le long de la périphérie, chaque arc de cercle ayant sa concavité tournée vers le haut, et une pluralité correspondante, notamment quatre, de tronçons de bord formant méplats, notamment horizontaux rectilignes, disposés en alternance avec les tronçons en arc de cercle.

4. Assemblage suivant l'une des revendications 1 à 3, **caractérisé en ce que** la plage inférieure de longitudes et la plage supérieure de longitudes sont distinctes l'une de l'autre, à savoir ne se chevauchent pas, par exemple la première plage étant égale à [60°,70°] et la deuxième plage étant égale à [75°, 85°].

5. Assemblage suivant l'une des revendications 1 à 4, **caractérisé en ce qu'**au moins une fenêtre de traversée a une forme triangulaire, dont une pointe est orientée vers le pôle supérieur de l'implant.

6. Assemblage suivant la revendication 5, **caractérisé en ce que** la au moins une fenêtre de traversée de forme triangulaire est une fenêtre supérieure.

7. Assemblage suivant l'une des revendications précédentes, **caractérisé en ce que** le corps de base est une sphère tronquée sur le dessus, notamment suivant un plan de coupe parallèle au plan équatorial, notamment sur une plage de longitudes comprises entre 60° à 70° et 90°.

8. Assemblage suivant l'une des revendications précédentes, **caractérisé en ce que** l'élément postérieur en forme de plaque est concave avec sa concavité tournée vers le bas, notamment en forme de calotte sphérique, notamment en ayant un rayon de courbure sensiblement égal au rayon de la partie sphérique de la sphère tronquée.

9. Assemblage suivant l'une des revendications précédentes, **caractérisé en ce que** les deux matériaux respectivement pour le corps de base et l'élément postérieur, sont à base d'une même matière, notamment thermoplastique, notamment le polyéthylène (PE), notamment le Polyéthylène Haute Densité (PEHD) ou le Polyéthylène de masse molaire très élevée (UHMWPE), mais en ayant des grains de tailles différentes, notamment compris entre 1 mm et 1,5 mm pour le corps de base et inférieur à 1,1 mm pour l'élément postérieur.

10. Implant orbital d'un assemblage suivant l'une des revendications 1 à 9, de forme sensiblement sphérique destiné à remplacer un globe oculaire après énucléation et/ou éviscération est constitué d'un corps (1) de base en un matériau poreux, notamment qui favorise la colonisation par l'organisme, et un élément (2) postérieur ou arrière à fenêtres traversantes recouvrant une partie du corps de base, l'élément postérieur comportant une pluralité de fenêtres traversantes pour le passage de fils de suture pour la suture des muscles oculomoteurs dans et hors de l'espace défini entre le corps de base et l'élément postérieur, et en définissant pour l'implant de forme sensiblement sphérique un repère passant par son centre, un axe vertical Z correspondant à l'axe antérieur-postérieur et deux axes XY dans le plan équatorial, repère par rapport auquel on définit les angles de latitude et de longitude d'un point quelconque à la surface de l'implant, l'angle de longitude du pôle le plus postérieur ou arrière, dit pôle supérieur, étant de 90° tandis que l'angle de longitude d'un point à l'équateur étant de 0°, **caractérisé en ce que** l'élément (1) à fenêtre comprend au moins une première fenêtre (7) traversante inférieure, qui s'étend sur une première plage inférieure de longitudes, et au moins une deuxième fenêtre (9) supérieure, qui s'étend sur une deuxième plage supérieure de longitudes, et il existe au moins un méridien (M) s'étendant de l'équateur au pôle qui traverse ou passe par les deux fenêtres (7) inférieure et ( 9) supérieure, la ou les fenêtre(s) (7) de traversée inférieures ont une forme oblongue ou allongée, un ou des bords inférieurs étant le ou les bords en longueur et s'étendant le long d'un bord périphérique de la surface extérieure du corps de base.

11. Implant suivant la revendication 10, **caractérisé en ce que** la plage inférieure de longitudes et la plage supérieure de longitudes sont distinctes l'une de l'autre, à savoir ne se chevauchent pas, par exemple la première plage étant égale à [60°,70°] et la deuxième plage étant égale à [75°, 85°].

12. Implant suivant la revendication 10 ou 11, **caractérisé en ce qu'**au moins une fenêtre de traversée a une forme triangulaire, dont une pointe est orientée vers le pôle supérieur de l'implant.

13. Implant suivant la revendication 12, **caractérisé en ce que** la au moins une fenêtre de traversée de forme triangulaire est une fenêtre supérieure.

14. Implant suivant les revendications 10 à 13, **caractérisé en ce que** le corps de base est une sphère tronquée sur le dessus, notamment suivant un plan de coupe parallèle au plan équatorial, notamment sur une plage de longitudes comprises entre 60° à 70° et 90°.

15. Implant suivant les revendications 10 à 14, **caractérisé en ce que** l'élément postérieur en forme de plaque est concave avec sa concavité tournée vers le bas, notamment en forme de calotte sphérique, notamment en ayant un rayon de courbure sensiblement égal au rayon de la partie sphérique de la sphère tronquée.

## Patentansprüche

1. Baugruppe, umfassend ein im Wesentlichen kugelförmiges Orbitaimplantat, das dazu bestimmt ist, nach Enukleation und/oder Eviszeration einen Augapfel zu ersetzen, und mindestens einen Nähfaden, wobei das Implantat aus einem Grundkörper (1) aus porösem Material, insbesondere solches, das die Besiedelung durch Organismen fördert, und einem hinteren oder rückseitigen Element (2) mit durchgehenden Fenstern, die einen Teil des Grundkörpers bedecken, besteht, wobei das hintere Element eine Vielzahl durchgehender Fenster für den Durchgang von Nähfäden zum Nähen der Augenmuskeln in und ausserhalb von dem zwischen dem Grundkörper und dem hinteren Element definierten Raum umfasst, und für das im Wesentlichen kugelförmige Implantat ein durch seinen Mittelpunkt verlaufendes Bezugssystem definiert wird, eine der Anterior-Posterior-Achse entsprechende vertikale Achse Z und zwei Achsen XY in der Äquatorialebene, ein Bezugssystem, hinsichtlich welchem die Breiten- und Längenwinkel eines beliebigen Punktes auf der Oberfläche des Implantats festgelegt werden, wobei der Längenwinkel des hintersten oder rückseitigsten Pols, oberer Pol genannt, 90° beträgt, während der Längenwinkel eines Punktes am Äquator 0° beträgt, **dadurch gekennzeichnet, dass** das Element (1) mit Fenster mindestens ein erstes unteres durchgehendes Fenster (7) hat, das sich auf einem ersten unteren Längenbereich erstreckt, und mindestens ein zweites oberes Fenster (9), das sich auf einem zweiten oberen Längenbereich erstreckt, und es mindestens einen sich vom Äquator zum Pol erstreckenden Meridian (M) gibt, der die beiden Fenster, das untere (7) und obere (9), durchquert oder passiert.

2. Baugruppe nach Anspruch 1, **dadurch gekennzeichnet, dass** das oder die untere(n) Durchgangsfenster (7) eine längliche oder gestreckte Form haben, wobei ein unterer Rand oder Ränder den Rand oder die Ränder in der Länge bilden und sich entlang eines umlaufenden Randes der Aussenfläche des Grundkörpers erstrecken.

3. Baugruppe nach Anspruch 2, **dadurch gekennzeichnet, dass** der umlaufende Rand, entlang welchem sich die unteren Ränder der unteren Durchgangsfenster erstrecken, durch eine Vielzahl von Kreisbogenabschnitten, insbesondere vier, definiert ist, die gleichmässig winkelförmig entlang des Umfangs verteilt sind, wobei jeder Kreisbogen seine Konkavität nach oben gerichtet hat, und eine entsprechende Vielzahl, insbesondere vier, von Randabschnitten, die Abflachungen bilden, insbesondere horizontal geradlinige, die alternierend zu den Kreisbogenabschnitten angeordnet sind.

4. Baugruppe nach einem der Ansprüche 1 bis 3, **dadurch gekennzeichnet, dass** der untere Längenbereich und der obere Längenbereich voneinander verschieden sind, das heisst, sich nicht überschneiden, zum Beispiel beträgt der erste Bereich gleich [60°,70°] und der zweite Bereich beträgt gleich [75°,85°].

5. Baugruppe nach einem der Ansprüche 1 bis 4, **dadurch gekennzeichnet, dass** mindestens ein Durchgangsfenster eine dreieckige Form hat, dessen eine Spitze zum oberen Pol des Implantats ausgerichtet ist.

6. Baugruppe nach Anspruch 5, **dadurch gekennzeichnet, dass** das mindestens eine dreieckige Durchgangsfenster ein oberes Fenster ist.

7. Baugruppe nach einem der vorstehenden Ansprüche, **dadurch gekennzeichnet, dass** der Grundkörper eine oben abgestumpfte Kugel ist, insbesondere entlang einer zur Äquatorialebene parallelen Schnittebene, insbesondere über einen Längenbereich zwischen 60° bis 70° und 90°.

8. Baugruppe nach einem der vorstehenden Ansprüche, **dadurch gekennzeichnet, dass** das hintere plattenförmige Element konkav ist, mit seiner nach unten gerichteten Konkavität, insbesondere in Kugelkalottenform, insbesondere mit einem Krümmungsradius, der im Wesentlichen gleich dem Radius des kugelförmigen Teils der abgestumpften Kugel ist.

9. Baugruppe nach einem der vorstehenden Ansprüche, **dadurch gekennzeichnet, dass** die beiden Materialien jeweils für den Grundkörper und das hintere Element auf demselben Material basieren, insbesondere Thermoplast, insbesondere Polyethylen (PE), insbesondere Polyethylen hoher Dichte (PE-HD) oder Polyethylen mit ultrahohem Molekulargewicht (UHMWPE), aber mit Körnern von unterschiedlicher Grösse, insbesondere zwischen 1 mm und 1,5 mm für den Grundkörper und weniger als 1,1 mm für das hintere Element.

10. Orbitaimplantat einer Baugruppe nach einem der Ansprüche 1 bis 9, im Wesentlichen kugelförmig, das dazu bestimmt ist, nach Enukleation und/oder Eviszeration einen Augapfel zu ersetzen, bestehend aus einem Grundkörper (1) aus porösem Material, insbesondere solchem, das die Besiedelung durch Organismen fördert, und einem hinteren oder rückseitigen Element (2) mit durchgehenden Fenstern, die einen Teil des Grundkörpers bedecken, wobei das hintere Element eine Vielzahl von durchgehenden Fenstern für den Durchgang von Nähfäden zum Nähen der Augenmuskeln in und ausserhalb von dem zwischen dem Grundkörper und dem hinteren Element definierten Raum umfasst, und für das im Wesentlichen kugelförmige Implantat ein durch seinen Mittelpunkt verlaufendes Bezugssystem definiert wird, eine der Anterior-Posterior-Achse entsprechende vertikale Achse Z und zwei Achsen XY in der Äquatorialebene, ein Bezugssystem, hinsichtlich welchem die Breiten- und Längenwinkel eines beliebigen Punktes auf der Oberfläche des Implantats festgelegt werden, wobei der Längenwinkel des hintersten oder rückseitigsten Pols, oberer Pol genannt, 90° beträgt, während der Längenwinkel eines Punktes am Äquator 0° beträgt, **dadurch gekennzeichnet, dass** das Element (1) mit Fenster mindestens ein erstes unteres durchgehendes Fenster (7) hat, das sich auf einem ersten unteren Längenbereich erstreckt, und mindestens ein zweites oberes Fenster (9), das sich auf einem zweiten oberen Längenbereich erstreckt, und es mindestens einen sich vom Äquator zum Pol erstreckenden Meridian (M) gibt, der die beiden Fenster, das untere (7) und obere (9), durchquert oder passiert, das oder die untere(n) Durchgangsfenster (7) eine längliche oder gestreckte Form haben, wobei ein unterer Rand oder Ränder den oder die Ränder in der Länge bilden und sich entlang eines umlaufenden Randes der Aussenfläche des Grundkörpers erstrecken.

11. Implantat nach Anspruch 10, **dadurch gekennzeichnet, dass** der untere Längenbereich und der obere Längenbereich voneinander verschieden sind, das heisst, sie überschneiden sich nicht, zum Beispiel beträgt der erste Bereich gleich [60° 70°] und der zweite Bereich beträgt gleich [75°, 85°].

12. Implantat nach Anspruch 10 oder 11, **dadurch gekennzeichnet, dass** mindestens ein Durchgangsfenster eine dreieckige Form hat, dessen eine Spitze zum oberen Pol des Implantats ausgerichtet ist.

13. Implantat nach Anspruch 12, **dadurch gekennzeichnet, dass** das mindestens eine dreieckige Durchgangsfenster ein oberes Fenster ist.

14. Implantat nach einem der Ansprüche 10 bis 13, **dadurch gekennzeichnet, dass** der Grundkörper eine oben abgestumpfte Kugel ist, insbesondere entlang einer zur Äquatorialebene parallelen Schnittebene, insbesondere über einen Längenbereich zwischen 60° bis 70° und 90°.

15. Implantat nach einem der Ansprüche 10 bis 14, **dadurch gekennzeichnet, dass** das hintere plattenförmige Element konkav ist, mit seiner nach unten gerichteten Konkavität, insbesondere in Kugelkalottenform, insbesondere mit einem Krümmungsradius, der im Wesentlichen gleich dem Radius des kugelförmigen Teils der abgestumpften Kugel ist.

## Claims

1. Assembly comprising an orbital implant of substantially spherical shape intended to replace an ocular globe after enucleation and/or evisceration and at least one stitch, the implant consisting of a basic body (1) made from a porous material, in particular one that encourages colonisation by organisms, and a posterior or rear element (2) with through-windows covering a part of the basic body, the posterior element comprising a number of through-windows for the passage of stitches for suturing the oculomotor muscles inside and outside the space between the basic body and the posterior element, and in defining for the implant of substantially spherical shape a reference axis passing through its centre, a vertical axis Z corresponding to the anterior-posterior axis and two axes XY in the equatorial plane, a reference frame in relation to which the angles of latitude and longitude of any point on the surface of the implant are defined, the angle of longitude of the rearmost or posterior pole, the said upper pole, being at 90° while the angle of longitude of a point on the equator being 0°, **characterised in that** element (1) with windows comprises at least one first lower through-window (7), that extends for a first lower range of longitudes, and at least one second upper window (9), that extends for a second upper range of longitudes and there exists at least one meridian (M) extending from the equator to the pole that crosses or passes through the two lower and upper windows( 7, 9).

2. Assembly according to claim 1, **characterised in that** the lower through-window(s) (7) have an oblong or elongated shape, one or two lower edges being the lengthwise edge(s) and extending along a surrounding edge of the exterior surface of the basic body.

3. Assembly according to claim 2, **characterised in that** the surrounding edge along which the lower edges of the lower through-windows extend is defined by a number of circular arc sections, in particular four, distributed at equal angles around the periphery, each circular arc with its concave side facing upwards, and a corresponding number, in particular four, of edge sections forming flats, in particular rectilinear horizontal, located alternately with the circular arc sections.

4. Assembly according to one of claims 1 to 3, **characterised in that** the lower range of longitudes and the upper range of longitudes are distinct from each other, that is they do not overlap, for example the first range being equal to [60°,70°] and the second range being equal to [75°, 85°].

5. Assembly according to one of claims 1 to 4, **characterised in that** at least one through-window has a triangular shape, one apex of which is oriented towards the upper pole of the implant.

6. Assembly according to claim 5, **characterised in that** the at least one through-window of triangular shape is an upper window.

7. Assembly according to one of the preceding claims, **characterised in that** the basic body is a sphere truncated at the top, in particular along a cutting plane parallel to the equatorial plane, in particular over a range of longitudes of between 60° to 70° and 90°.

8. Assembly according to one of the preceding claims, **characterised in that** the posterior element in the form of a plate is concave with its concave side facing downwards, in particular in the form of a spherical cap, in particular having a radius of curvature substantially equal to the radius of the spherical part of the truncated sphere.

9. Assembly according to one of the preceding claims, **characterised in that** the two materials for the basic body and the posterior element respectively, are based on the same material, in particular thermoplastic, in particular polyethylene (PE), in particular High Density Polyethylene (PEHD) or Polyethylene of very high molar mass (UHMWPE), but having grains of different sizes, in particular between 1 mm and 1.5 mm for the basic body and less than 1.1 mm for the posterior element.

10. Orbital implant of an assembly as defined in the claims 1 to 9, of substantially spherical shape intended to replace an ocular globe after enucleation and/or evisceration constituted of a basic body (1) made from a porous material, in particular one that encourages colonisation by organisms, and a posterior or rear element (2) with through-windows covering a part of the basic body, the posterior element comprising a number of through-windows for the passage of stitches for suturing the oculomotor muscles inside and outside the space between the basic body and the posterior element, and defining for the implant of substantially spherical shape a reference axis passing through its centre, a **vertical** axis Z corresponding to the anterior-posterior axis and two axes XY in the equatorial plane, a reference frame in relation to which the angles of latitude and longitude of any point on the surface of the implant are defined, the angle of longitude of the rearmost or posterior pole, called the upper pole, being 90° while the angle of longitude of a point on the equator being 0°, **characterised in that** the element (1) with windows comprises at least one first lower through-window (7), that extends for a first lower range of longitudes, and at least one second upper window (9), that extends for a second upper range of longitudes, and there exists at least one meridian (M) extending from the equator to the pole that crosses or passes through the two lower windows (7) and upper windows (9), the lower through-window(s) (7) have an oblong or elongated shape, one or more lower edges being the lengthwise edges and extend along a surrounding edge of the exterior surface of the basic body.

11. Implant according to claim 10, **characterised in that** the lower range of longitudes and the upper range of longitudes are distinct from each other, that is they do not overlap, for example the first range being equal to [60°,70°] and the second range being equal to [75°, 85°].

12. Implant according to claim 10 or 11, **characterised in that** the at least one through-window has a triangular shape, one apex of which is oriented towards the upper pole of the implant.

13. Implant according to claim 12, **characterised in that** the at least one through-window of triangular shape is an upper window.

14. Implant according to claims 10 to 13, **characterised in that** the basic body is a sphere truncated on the top, in particular along a cutting plane parallel to the equatorial plane, in particular for a range of longitudes of between 60° to 70° and 90°.

15. Implant according to claims 10 to 14, **characterised in that** the posterior element in the form of a plate is concave with its concave side facing downwards, in particular in the form of a spherical cap, in particular in having a radius of curvature substantially equal to the radius of the spherical part of the truncated sphere.
